(19) Europäisches Patentamt

European Patent Office

Office européen des brevets

(11) Veröffentlichungsnummer: **0 502 917 B1**

(12) **EUROPÄISCHE PATENTSCHRIFT**

(45) Veröffentlichungstag der Patentschrift: **14.06.95**

(21) Anmeldenummer: **90917724.8**

(22) Anmeldetag: **19.11.90**

(86) Internationale Anmeldenummer:
**PCT/EP90/01976**

(87) Internationale Veröffentlichungsnummer:
**WO 91/08192 (13.06.91 91/13)**

(51) Int. Cl.⁶: $C07C$ **69/54**, $C07C$ 67/56

(54) **VERFAHREN ZUM INHIBITORAUSTAUSCH IN RADIKALISCH REAKTIVEN OLEFINISCH UNGESÄTTIGTEN SYSTEMEN.**

(30) Priorität: **27.11.89 DE 3939162**

(43) Veröffentlichungstag der Anmeldung:
**16.09.92 Patentblatt 92/38**

(45) Bekanntmachung des Hinweises auf die Patenterteilung:
**14.06.95 Patentblatt 95/24**

(84) Benannte Vertragsstaaten:
**BE DE ES FR GB GR IT NL**

(56) Entgegenhaltungen:
**EP-A- 0 376 089**
**DE-A- 2 149 530**
**FR-A- 1 361 512**

(73) Patentinhaber: **Henkel Kommanditgesellschaft auf Aktien**

**D-40191 Düsseldorf (DE)**

(72) Erfinder: **SITZ, Hans-Dieter**
**Widdeshovener Strasse 48**
**W-4049 Rommerskirchen (DE)**
Erfinder: **RITTER, Wolfgang**
**Am Bandenfeld 74**
**W-5657 Haan (DE)**

(74) Vertreter: **von Kreisler, Alek, Dipl.-Chem. et al**
**Patentanwälte**
**von Kreisler-Selting-Werner**
**Postfach 10 22 41**
**D-50462 Köln (DE)**

EP 0 502 917 B1

Rank Xerox (UK) Business Services
(3.10/3.09/3.3.3)

**Beschreibung**

Die Erfindung beschreibt eine technische Lehre, durch die wichtige Verbesserungen auf dem Gebiet der gezielten Inhibierung radikalisch reaktiver, olefinisch ungesättigter Systeme möglich wird. Die Erfindung will insbesondere einen neuen Weg aufzeigen, der die Möglichkeit eröffnet, die in diesen Systemen zur Stabilisierung eingesetzten Radikalinhibitoren nach Art und Menge frei wählbar einzustellen und diese freie Wählbarkeit auch gerade dann sicherzustellen, wenn bisher bekannte Technologien zur Lösung dieser Aufgabe versagen.

Die DE-OS-2 149 530 will einen verbesserten Weg aufzeigen, Polymerisationsinhibitoren aus ethylenisch ungesättigen monomeren polymerisierbaren Verbindungen zu entfernen. Angesprochen sind zunächst die drei Möglichkeiten der Destillation, der Extraktion mit Hilfe von Ionenaustauschharzen, und die wässrig alkalische Wäsche unter Einsatz verdünnter Lösungen eines Alkalihydroxids. Als Verbesserung wird in dieser Druckschrift vorgeschlagen, die alkalische Wäsche mit vergleichsweise konzentrierten wässrigen Alkalilösungen derart vorzunehmen, daß diese fließfähigen Neutralisationsmittel unter Ausbildung einer großen Oberfläche den den Inhibitor enthaltenden Monomeren angeboten werden. Das kann entweder durch heftiges Rühren erfolgen oder dadurch, daß man feinverteilte feste Stoffe zugibt und damit die wässrige Alkalihydroxidlösung auf deren Oberfläche spreitet. Auch die Kombination der beiden Maßnahmen ist möglich. Die FR-A-1 361 512 schlägt zur Lösung des gleichen technischen Problems vor, ausgewählte und in bestimmter Weise vorbehandelte Feststoffe den Inhibitor enthaltenden Reaktionsmassen zuzusetzen. Konkret sind die folgenden Feststoffe als Zusatzmittel genannt: ausgewählte Aluminiumoxide, die bei Temperaturen zwischen 275° und 350°C für mehrere Stunden aktiviert worden sind. Genannt sind weiterhin Kieselsäureverbindungen, insbesondere Alkalisilikate wie Natriumetasilikat, die zunächst zu trocknen und dann zu Körnern eines mittleren Durchmessers von 0,1 bis 2,0 mm aufzuarbeiten sind. Dieses Material muß für vier Stunden auf Temperaturen zwischen 90° und 110°C erhitzt werden. Schließlich sind als eine weitere Möglichkeit molekulare Siebe vom Zeolith-Typ benannt. Auch diese festen Hilfsstoffe bedürfen der Vorbehandlung unter Schutzgas für den Zeitraum von vier bis fünf Stunden im Temperaturbereich von 300° bis 400°C.

Die Mitverwendung von Inhibitoren bei der Herstellung, der Lagerung und der Verarbeitung radikalisch sensitiver olefinisch ungesättigter Verbindungen bzw. Systeme ist chemisches Allgemeingut. Die Inhibitoren bzw. Inhibitorsysteme sollen die radikalische Reaktionsauslösung zum unerwünschten Zeitpunkt verhindern, auf der anderen Seite muß die Inhibitorwirkung beim Einsatz dieser Systeme durch gezielte radikalische Reaktionsauslösung überwunden werden. Je nach Herstellungsbedingungen der olefinisch ungesättigten Verbindungen bzw. entsprechender Systeme, der Reaktivität der betroffenen Verbindungen bzw. Molekülbestandteile den Bedingungen der Lagerung und schließlich den vorgegebenen Einsatzbedingungen können Schwierigkeiten auftreten, das dargestellte breite Anforderungsprofil zur Stabilitätsregulierung durch Radikalinhibitoren mit nur einem ausgewählten Inhibitor bzw. Inhibitorsystem abzudecken. Man kann dementsprechend zwischen "Herstellungsinhibitoren" und "Applikationsinhibitoren" unterscheiden, die nach Art und/oder Menge ungleich sein können. So kann es beispielsweise zweckmäßig sein, bei der Herstellung der Reaktivsysteme vergleichsweise starke Inhibitoren und/oder vergleichsweise erhöhte Inhibitormengen (Herstellungsinhibitoren) einzusetzen, während die Lagerung und/oder Verarbeitung des Reaktivsystemes dann nach Art und Menge nur noch vergleichsweise schwächer wirksame Inhibitoren bzw. Inhibitorsysteme (Applikationsinhibitoren) erfordern. In zahlreichen Fällen ist ein vergleichsweise problemloser Austausch der Herstellungsinhibitoren gegen die Applikationsinhibitoren möglich. Als Beispiel sei die destillative Reinigung von Stoffgemischen genannt, die vergleichsweise schwerflüchtige Inhibitoren in Abmischung mit destillierbaren olefinisch Ungesättigten Komponenten enthalten, so daß die destillative Abtrennung der inhibitorfreien olefinisch ungesättigten Verbindung möglich wird. Das so gewonnene Reinprodukt kann dann in vorbestimmbarer Weise mit dem Applikationsinhibitor versetzt werden.

Die Praxis kennt allerdings zahlreiche Fälle, in denen eine solche problemlose Auftrennung des aus der Herstellung primär anfallenden Stoffgemisches aus Herstellungsinhibitor und radikalisch reaktiver Verbindung nicht gegeben ist. Ein solcher Fall liegt beispielsweise vor, wenn eine destillative Trennung des primär anfallenden inhibierten Stoffgemisches - beispielsweise wegen zu hoher Siedepunkte der ungesättigten Komponenten oder anderen Gründen - ausscheidet. In diesen und vergleichbaren Fällen wird in der Regel der Herstellungsinhibitor zwangsläufig auch zum Applikationsinhibitor. Durch diese Verknüpfung ergeben sich häufig unerwünschte Bindungen für die Technologie der Herstellungsstufe der radikalisch reaktiven Systeme an die technologischen Erfordernisse an das System einschließlich seines Inhibitorgehaltes in der praktischen Anwendung.

Die Erfindung geht von der Aufgabe aus, einen neuen Weg aufzuzeigen, wie eine solche unerwünschte Bindung der unterschiedlichen Gesetzmäßigkeiten unterliegenden Technologien der jeweiligen Stufe bezüglich der Beschaffenheit des jeweils vorliegenden Inhibitors aufgelöst werden kann. Die Erfindung will insbesondere die Möglichkeit schaffen, in radikalisch reaktiven Systemen der hier betroffenen Art die Beschaffenheit des Applikationsinhibitors nach Art und Menge frei wählbar einzustellen, ohne dabei Rücksicht auf die Besonderheiten bei der Herstellung der radikalisch sensitiven Systeme nehmen zu müssen. Zur technischen Lösung dieser Aufgabe schlägt die Erfindung den Einsatz einer Gruppe ausgewählter Inhibitoren als Herstellungsinhibitor vor, die durch einfache Abreaktion mit den im nachfolgenden beschriebenen Hilfsstoffen gebunden und in dieser Form aus dem System entfernt werden können, während dann die Zugabe von Applikationsinhibitoren in freier Wahl nach Art und/oder Menge möglich wird.

Gegenstand der Erfindung ist dementsprechend ein Verfahren zum teilweisen oder vollständigen Austausch der Radikalinhibitoren (Herstellungsinhibitor) aus ihren Abmischungen mit radikalisch reaktiven, insbesondere polymerisierbaren und/oder vernetzbaren 1- und/oder mehrfach olefinisch ungesättigten Verbindungen gegen nach Art und Menge frei bestimmbare radikalische Inhibitoren oder Inhibitor-Systeme (Applikationsinhibitor), wobei das Verfahren dadurch gekennzeichnet ist, daß man von radikalisch reaktiven Abmischungen ausgeht, die als Herstellungsinhibitor entsprechende Verbindungen des Phenoltyps mit zur Salzbildung befähigten Hydroxylgruppen enthalten, dieses Einsatzgemisch als Flüssigphase einer Behandlung mit festen Oxiden, Carbonaten und/oder Hydroxiden der Alkali- und/oder Erdalkalimetalle, die auch in Abmischung mit weiteren zur Salzbildung befähigten basischen Metalloxidverbindungen vorliegen können, unterwirft und dabei in vorbestimmbarer Weise den Herstellungsinhibitor ganz oder teilweise an die Feststoffphase bindet, die gebildete Feststoffphase von der Flüssigphase abtrennt und in dieser Flüssigphase den Gehalt an Applikationsinhibitor nach Art und Menge geregelt einstellt.

Die erfindungsgemäße Lehre baut damit auf zwei wesentlichen Elementen auf: Als Herstellungsinhibitoren werden ausgewählte Verbindungen dieses Typs mit zur Salzbildung befähigten Hydroxylgruppen vorgesehen. Ihre Entfernung aus den radikalisch reaktiven Systemen wird durch Behandlung mit ausgewählten Feststoffreaktanten unter Anbindung der Herstellungsinhibitoren an diese Feststoffe möglich, während das zu inhibierende System in der Flüssigphase gehalten wird. Durch eine einfache Phasentrennung gelingt dann die Abreicherung und auch vollständige Entfernung der Herstellungsinhibitoren aus dem Einsatzgemisch. Hierdurch wird die freie Wahl des Applikationsinhibitors nach Art und/oder Menge möglich. Das erfindungsgemäße Verfahren eignet sich insbesondere für solche radikalisch reaktiven Stoffgemische, die auf destillativem Wege nicht oder nicht befriedigend von ihrem Gehalt an Herstellungsinhibitoren befreit werden können.

Phenolverbindungen mit zur Salzbildung befähigten freien Hydroxylgruppen sind eine in der Polymerisationstechnik bekannte Klasse von Verbindungen mit ausgeprägter Inhibitorwirksamkeit gegen radikalische Reaktionsauslösung in olefinisch ungesättigten Systemen. Besonders wirksame Vertreter finden sich in der Klasse der Hydrochinonverbindungen, insbesondere der ringsubstituierten Hydrochinonverbindungen.

Es hat sich gezeigt, daß unter den im nachfolgenden noch im einzelnen diskutierten bevorzugten Reaktionsbedingungen für die Umsetzung solcher Inhibitoren vom Phenoltyp bzw. Hydrochinontyp mit den festen Oxiden und/oder Hydroxiden der Erdalkalimetalle mehr oder weniger stark ausgeprägte Unterschiede in der jeweils stofftypischen Reaktionsbereitschaft zur Salzbildung unter Anbindung des Inhibitors an das in Feststoffphase eingesetzte Fällungsmittel vorliegen. Insbesondere Alpha-substituierte Hydrochinonverbindungen und hier wiederum Dialkyl-substituierte Hydrochinone sind besonders reaktiv und eignen sich dementsprechend zum Einsatz als Herstellungsinhibitor im erfindungsgemäßen Verfahren besonders. Hier liegt eine Inhibitorklasse vor, die sich durch hohe Hemmwirkung gegen unerwünschte radikalische Polymerisation auszeichnet, so daß eine für die praktischen Bedürfnisse befriedigende Hemmwirkung gegen unerwünschte radikalische Abreaktion in der Herstellungsphase eingestellt werden kann. Besonders stark hemmwirksame Inhibitoren sind beispielsweise Dialkyl-substituierte Hydrochinone und hier insbesondere das 2,5-Di-tert.-butylhydrochinon, das in praktisch frei wählbaren Mengen in der Stufe der Herstellung der Reaktivsysteme zur Stabilisierung eingesetzt werden kann. Durch die erfindungsgemäß nachgeschaltete Abtrennung solcher salzbildenden Inhibitoren über ihre wenigstens anteilsweise Anbindung an ein festes Fällungsmittel und nachfolgende Phasentrennung wird es möglich, die beispielsweise radikalisch stark inhibierenden Herstellungsinhibitoren nach Art und/oder Menge gegen schwächer inhibierende Applikationsinhibitoren auszutauschen.

Die der Erfindung zugrundeliegenden Prinzipien werden im nachfolgenden beispielhaft anhand einer ausgewählten Stoffklasse beschrieben, bei deren Herstellung die Mitverwendung der erfindungsgemäß vorgeschlagenen Maßnahmen besonders interessant ist. Für den Fachmann sofort einleuchtend gilt allerdings, daß der Umfang des Anwendungsgebietes der Erfindung sich nicht auf diese bestimmte Stoffklasse einschränkt. Die neu vorgeschlagenen Arbeitsmaßnahmen sind in beliebigen Anwendungsfällen vergleichbarer technologisch vorgegebener oder frei gewählter Problemstellung einsetzbar.

Schwerflüchtige polyfunktionelle Ester der Acrylsäure und/oder der Methacrylsäure mit mehrwertigen Alkoholen - im folgenden auch als (Meth)acrylsäureester bzw. Poly(meth)acrylsäureester bezeichnet - werden bekanntlich durch katalytische Umsetzung der Reaktanten unter Zusatz von Polymerisationsinhibitoren zum Reaktionsgemisch hergestellt. (Meth)acrylsäureester dieser Art finden zunehmende Bedeutung als hochreaktive Bestandteile in beispielsweise strahlenhärtenden Systemen. Die hier zugrundeliegenden polyfunktionellen Alkohole sind beispielsweise 2- bis 4-wertige aliphatische gesättigte Alkohole und/oder deren Oxalkylierungsprodukte. Polyfunktionelle (Meth)acrylsäureester der genannten Art können beispielsweise als Lackrohstoffe für die Elektronenstrahlhärtung oder als Bestandteil von UV-Lichthärtenden Druckfarben oder entsprechenden Überzugslacken, in Spachteln, Form- oder Gußmassen sowie in Klebstoffen, insbesondere anaerob härtenden Klebstoffen Verwendung finden. Wegen ihrer vergleichsweise hohen Reaktivität ist die Mitverwendung von Inhibitoren erforderlich, die nach Art und/oder eingesetzter Menge die sichere Inhibierung des der Veresterung unterworfenen Systems gewährleisten und das beispielsweise auch dann noch, wenn auf die Mitverwendung inerter Lösungsmittel verzichtet und stattdessen die Veresterungsreaktion in Substanz durchgeführt werden soll. Es leuchtet sofort ein, daß hier beträchtliche Leistungsanforderungen an den Herstellungsinhibitor gestellt werden müssen.

Die praktische Verwertung solcher schwerflüchtigen und destillativ nicht zu reinigenden Systeme kann dann aber durch die hohe, bei der Herstellung erforderliche Inhibitorwirkung behindert werden. Die Erfindung schafft die Möglichkeit, diese anwendungstechnische Behinderung zu beseitigen.

Ein anderer sofort einsichtiger Fall für den Einsatz des erfindungsgemäßen Verfahrens liegt dann vor, wenn der Rahmen rein technischer bzw. technologischer Überlegungen insbesondere durch den beabsichtigten Einsatz der Reaktivsysteme gesprengt wird. Dies ist immer dann der Fall, wenn die Reaktivsysteme in der Anwendung einer Überprüfung ihrer physiologischen Verträglichkeit bedürfen, wie es beispielsweise im Rahmen der Klebstofftechnologie im lebenden Organismus der Fall ist. Hochwirksame Inhibitoren von der Art des 2,5-Di-tert.-butylhydrochinons sind hier aus physiologischen Überlegungen heraus nicht die Komponenten erster Wahl. Es werden Klebstoffe bzw. Klebstoffsysteme gesucht, die physiologisch besser verträgliche Applikationsinhibitoren enthalten. Erfindungsgemäß wird ein solcher Austausch von Herstellungsinhibitoren begrenzter physiologischer Verträglichkeit gegen physiologisch besser verträgliche Applikationsinhibitoren möglich. Ein besonders geeigneter Inhibitor ist hier das Vitamin E, dessen Verwendung für Zwecke der hier genannten Art im einzelnen beschrieben wird in der DE-A1 39 39 161.

Besondere Bedeutung kommt der Verwendung von feinteiligen, insbesondere feinpulvrigen Oxiden und/oder Hydroxiden von Calcium und/oder Magnesium zur Bindung des Herstellungsinhibitors zu. Hierbei gilt, daß von diesen beiden Erdalkalimetallen den entsprechenden Verbindungen des Calciums wiederum besondere Bedeutung zukommt. In einer bevorzugten Ausführungsform der Erfindung werden feinteilige Feststoffe in die Stufe der Inhibitorbindung eingeführt, die wenigstens anteilsweise Calciumverbindungen der genannten Art enthalten. In einer besonders wichtigen Ausführungsform wird Calciumhydroxid $Ca(OH)_2$ und/oder gebrannter Kalk CaO eingesetzt. Insbesondere feinteiliges Calciumhydroxid ermöglicht häufig eine optimale Entfernung des Herstellungsinhibitors aus dem Reaktionsgemisch.

Durch Optimierung der Verfahrensparameter in der Stufe der Bindung des Herstellungsinhibitors ist es möglich, in vorbestimmbarer Weise das Ausmaß der Entfernung des unerwünschten Inhibitors oder unerwünschter überschüssiger Anteile des Inhibitors durchzuführen. Die erfindungsgemäß ausgewählten Feststoffsysteme auf Basis der Oxide und/oder Hydroxide der Erdalkalimetalle, insbesondere des Magnesiums und/oder des Calciums können bei vergleichsweise hochreaktiven olefinisch ungesättigten Systemen im Bereich der Raumtemperatur oder auch noch bei schwach erhöhten Temperaturen zur Auslösung einer anionischen Polymerisation und damit zur Vergelung des Reaktionsproduktes führen. Erfindungsgemäß ist es dementsprechend bevorzugt, die Bindung des Herstellungsinhibitors bei Temperaturen oberhalb 50 °C durchzuführen, wobei sich als besonders interessanter Temperaturbereich die Spanne von etwa 60 bis 110 °C und insbesondere der Bereich von etwa 70 bis 90 °C erwiesen haben. Temperaturen dieses Arbeitsbereiches bevorzugen die salzbildende Inhibitorbindung gegenüber der anionischen Polymerisationsauslösung.

Aus Gründen der Wirtschaftlichkeit aber auch aus Gründen des optimalen Schutzes des an sich labilen Reaktionsgemisches wird das feste basische Reaktionshilfsmittel vorzugsweise nur in beschränktem stöchiometrischen Überschuß eingesetzt. Die hinreichende Verweildauer des Feststoffes in Suspension im flüssigen Einsatzgut muß also ausreichend lang gewählt werden, um den erwünschten Kontakt zwischen festen und flüssigen bzw. gelösten Inhibitoren sicherzustellen. Darüber hinaus belegt sich die Außenfläche des individuellen Feststoffpartikels mit dem jeweils entstehenden Salz. Das tiefere Eindringen der reaktiven Komponenten in den Kern des Feststoffteilchens wird damit zusätzlich behindert.

Die Behandlungsdauer einer flüssigen, Herstellungsinhibitor enthaltenden reaktiven Phase im erfindungsgemäßen Sinne zur wenigstens anteilsweisen Inhibitorbindung kann über einen Zeitraum von einigen Stunden beispielsweise bis zu 5 Stunden, vorzugsweise bis zu 3 Stunden durchgeführt werden. Für eine Optimierung im Sinne des erfindunsgemäßen Verfahrens kann es allerdings zweckmäßig sein, Verfahrenstemperatur und Verfahrensdauer so aufeinander abzustimmen, daß zur Einstellung der erwünschten niederen Gehalte an Herstellungsinhibitor eine Behandlungsdauer unterhalb 1 Stunde und insbesondere unterhalb 45 Minuten möglich wird. In besonders bevorzugten Ausführungsformen werden die Arbeitsbedingungen so aufeinander abgestimmt, daß die angestrebten Restgehalte an Herstellungsinhibitor im Zeitraum bis etwa 30 Minuten eingestellt werden. Beim Arbeiten mit Calciumhydroxid können in der Regel optimale Werte im Temperaturbereich um ca. 80 °C bei einer Behandlungsdauer von etwa 10 bis 30 Minuten eingestellt werden.

Es hat sich weiterhin gezeigt, daß die Gegenwart von Wasser im zu behandelnden Reaktionsgut nachteilig sein kann. Auch vergleichsweise geringe Mengen wäßriger Basenlösungen der hier betroffenen Art wirken offenbar stärker auf das Reaktionsgut ein als das erfindungsgemäß eingesetzte feste Behandlungsmittel in Trockenform. Erfindungsgemäß ist dementsprechend zunächst einmal bevorzugt, das von Inhibitor wenigstens anteilsweise zu befreiende und als Flüssigphase einzusetzende Gut wenigstens weitgehend wasserfrei vorzulegen. Im` zu reinigenden Gut gegebenenfalls vorliegende Restwassermengen können in an sich bekannter Weise beispielsweise unter Mitverwendung von Hilfslösungsmitteln in azeotroper Destillation oder einfach durch Anlegen von Vakuum für einen hinreichend langen Zeitraum abgezogen werden.

Die Menge des in der Behandlungsstufe zugesetzten feinpulvrigen basischen Hilfsstoffes wird primär durch die insgesamt zu entfernenden Anteile an Herstellungsinhibitor im Rohprodukt bestimmt. Vorzugsweise wird dabei mit einem stöchiometrischen Überschuß der feinpulvrigen Hilfsmittel gearbeitet. Es kann in der Regel zweckmäßig sein, mit einem beschränkten Überschuß dieses festen Hilfsstoffes im Bereich bis zum etwa 10-fachen der stöchiometrisch benötigten Menge, vorzugsweise mit Anteilen bis zum etwa 5-fachen Überschuß der stöchiometrisch benötigten Menge zu arbeiten. Aber auch das Arbeiten mit vergleichsweise beschränktem Überschuß, z. B. im Bereich von wenigstens dem etwa 1,3-fachen bis zum etwa 3-fachen der stöchiometrisch benötigten Menge kann geeignet sein.

Reaktivmassen der hier betroffenen Art sind - wie aus den angegebenen Beispielen ersichtlich - häufig Ester oder vergleichbare Reaktionsprodukte, die durch Restgehalte von Katalysatoren insbesondere sauren Katalysatoren, Restsäuren aus der Synthese und dergleichen Verunreinigungen gekennzeichnet sind. Die erfindungsgemäße Behandlung mit den basischen Feststoffen der geschilderten Art kann dann gleichzeitig zur partiellen oder vollständigen Entfernung solcher sauren Anteile im Sinne einer Trockenneutralisation führen. Einzelheiten zur Trockenneutralisation radikalisch reaktiver Stoffgemische in Flüssigphase mit den festen bevorzugt feinteiligen Oxiden und/oder Hydroxiden der Erdalkalimetalle, die auch in Abmischung mit weiteren oxidischen Metallverbindungen vorliegen können, werden in der DE-A1- 39 39 163. ("Verbessertes Trockenneutralisationsverfahren für organische Flüssigphasen") der Anmelderin beschrieben. Geschildert wird dort die Lehre, daß man zur Gewinnung von Reinprodukten, die auch ohne Destillation niedrige Restsäurezahlen mit niedrigen Farbzahlen verbinden, die Neutralisation mit festen feinpulvrigen Oxiden und/oder Hydroxiden der Erdalkalimetalle als Trockenneutralisation durchführt und nachfolgend die organische Flüssigphase von der feinpulvrigen Feststoffphase abtrennt. Es gelingt auf diese Weise, unter den in der parallelen Patentanmeldung im einzelnen genannten Arbeitsbedingungen optimierte Produkteigenschaften einzustellen, insbesondere niedrige Restsäurezahlen mit niedrigen Farbwerten zu verbinden. Die Lehre der vorliegenden Erfindung sieht die Kombination dieser Reinigungsmaßnahmen mit der erfindungsgemäß im Vordergrund stehenden anteilsweisen oder vollständigen Entfernung der Herstellungsinhibitoren aus diesen Reaktionsmassen vor. Insbesondere in dieser Ausführungsform verdient ein zusätzlicher Gesichtspunkt besondere Erwähnung: Es hat sich als zweckmäßig erwiesen, die Reaktion des zu reinigenden Flüssiggutes mit der feinpulvrigen basischen Feststoffmasse selbst dann wenigstens anteilsweise unter verringertem Druck durchzuführen, wenn das eingesetzte zu neutralisierende und von Herstellungsinhibitor zu befreiende Flüssiggut an sich wasserfrei ist. Bei der Arbeitsweise unter verringertem Druck wird auch der im Rahmen der Neutralisation entstehende Wasseranteil aus dem Reaktionsgemisch abgezogen und damit eine in situ Bildung wäßrig-basischer Lösungsanteile bei der Neutralisation verhindert oder zumindest eingeschränkt. Es kann hier zweckmäßig sein, die Behandlung im Druckbereich von etwa 1 bis 150 mbar, z. B. bei etwa 20 bis 150 mbar durchzuführen, wobei das Arbeiten im Bereich von etwa 20 bis 100 mbar besonders bevorzugt sein kann. Auf diese Weise gelingt es, Restwassergehalte im Fertigprodukt auf Werte unter etwa 0,1 Gew.-% abzusenken, während bei der Trockenbehandlung praxisüblicher Rohprodukte aufgrund ihres Gehaltes an freien Säurekomponenten - insbesondere Katalysatoren, Restsäuren und dergleichen - nach Abschluß der Behandlung ohne Anlegen von Vakuum Restwassergehalte im Bereich von etwa 0,7 bis 0,9 Gew.-% liegen können. Es hat sich gezeigt, daß schon durch diese höheren Restwasser-Gehalte substantielle Verschlechterungen in der Farbzahl des gereinigten Endproduktes ausgelöst werden können.

Wie bereits angegeben, können die oxidischen bzw. hydroxidischen Erdalkaliverbindungen auch in Abmischung mit weiteren zur Salzbildung befähigten basischen Metalloxiden bzw. Metalloxidverbindungen zum Einsatz kommen. Ein besonders interessantes Beispiel ist hier der Hydrotalcit. Kombinationen aus Hydrotalcit und basischen Calciumverbindungen führen zu einer besonders guten Kombination der angestrebten Werte im Fertigprodukt. Zur Beschaffenheit des natürlich vorkommenden und/oder synthetisch herzustellenden Hydrotalcits wird auf die einschlägige Literatur verwiesen, vgl. hierzu beispielsweise R. Allmann et al. "Die Struktur des Hydrotalkits" N. Jahrb. Mineral. Monatsh. 1969, 544 bis 551.

Als Applikationsinhibitoren können dann nach Art und Menge freigewählte Inhibitorsysteme bzw. individuelle Inhibitorkomponenten zugesetzt werden, die auf die jeweiligen Anforderungen aus Lagerung und dem Einsatz der gereinigten Reaktivmassen abgestimmt sind. Wie bereits angegeben kann eine wichtige Ausführungsform darin liegen, daß man durch die erfindungsgemäße Reinigungsbehandlung radikalisch stark inhibierende Herstellungsinhibitoren nach Art und/oder Mengen gegen schwächer inhibierende Applikationsinhibitoren austauscht oder daß man Herstellungsinhibitoren begrenzter physiologischer Verträglichkeit gegen physiologisch besser verträgliche Applikationsinhibitoren austauscht.

Aus der umfangreichen Literatur zur Bedeutung, Ausgestaltung und Auswahl von Inhibitoren und Inhibitorsystemen der hier betroffenen Art, die als Applikationsinhibitoren Anwendung finden können, wird beispielsweise verwiesen auf Polymer Handbook 2. Ausgabe JOHN WILEY & SONS, New York 1979, Band 11, 53 bis 55 sowie auf Enzyc. Polym. Sci. Technol. 1967, Vol. 7, 644 bis 664.

**Beispiele**

Beispiel 1

In einem 1-Liter-Kolben wurden jeweils 8.00 g eines Veresterungsrohproduktes - bestehend aus 28 g Toluolsulfonsäure, 1,6 g 2,5-Di-tert.-butylhydrochinon,0,8 g Hydrochinon, 30,8 g Acrylsäure und 740,6 g Trimethylolpropan + 3 EO-triacrylat - eingewogen. Die Neutralisation des Rohproduktes erfolgte bei einer Temperatur von 80 °C unter Rühren und Durchleiten von 10 l Luft/h. Zur Neutralisation wurde die zweifach äquivalente Menge an Base - bezogen auf die Säurezahl des Rohproduktes (Säurezahl: 40 mg KOH/g Substanz) - eingesetzt.

Nach 1, 2 und 3 Stunden wurden jeweils ca. 260 g des neutralisierten Produktes entnommen und mittels Druckfilternutsche filtriert. Die Ergebnisse bezüglich des Inhibitorgehaltes und der Säurezahl sind in der Tabelle 1 zusammengefaßt.

## Tabelle 1

Inhibitorgehalte in Abhängigkeit vom Neutralisationsmittel und von der Neutralisationszeit

Ausgangsprodukt: Trimethylolpropan ü 3 EO-triacrylat

Säurezahl: 40 mg KOH/g

Inhibitoren: 2000 ppm 2,5-Di-tert.-butylhydrochinon (DTBHQ), 1000 ppm Hydrochinon (HQ)

| Temperatur: 80 °C | Neutralisationszeit min. | Inhibitorgehalte DTBHQ ppm | HQ ppm | Säurezahl mg KOH/g |
|---|---|---|---|---|
| LiOH | 60 | 1000 | 820 | 9,8 |
| (2fach äquival.) | 120 | 0 | 740 | 2,2 |
| | 180 | 0 | 670 | 1,3 |
| | | | | |
| NaOH | 60 | 1000 | 770 | 25,8 |
| (feinpulvrig) | 120 | 0 | 750 | 5,0 |
| (2fach äquival.) | 180 | 0 | 720 | 2,8 |
| | | | | |
| Ca(OH)2 | 60 | 0 | 670 | 0,9 |
| (2fach äquival.) | 120 | 0 | 590 | 0,2 |
| | 180 | 0 | 540 | 0,1 |
| | | | | |
| MgO | 60 | 890 | 930 | 5,8 |
| (2fach äquival.) | 120 | 850 | 930 | 5,6 |
| | 180 | 820 | 930 | 3,1 |
| | | | | |
| CaO | 60 | 1180 | 980 | 23,6 |
| (2fach äquival.) | 120 | 890 | 960 | 8,4 |
| | 180 | 0 | 950 | 3,2 |

Beispiel 2

1,0 kg eines Veresterungsproduktes (35 g p-Toluolsulfonsäure; 2 g 2,5-Di-tert.-butylhydrochinon; 35,9 g Acrylsäure, 927,1 g Trimethylolpropan + 3 EO-triacrylat; Säurezahl 38 mg KOH/g) wurden in einen 2-Liter-Reaktor eingewogen und bei 80 °C und 50 mbar unter Durchleiten von 20 l Luft/h neutralisiert. Bei den Neutralisationsmitteln handelt es sich um Ca(OH)$_2$, CaO sowie um Kombinationsanwendungen von schwächer und stärker basischen Substanzen.

| Ca(OH)$_2$: | 49,9 g (2fach äquivalent) |
|---|---|
| CaO: | 37,8 g (2fach äquivalent) |

| Mischungen (1 : 1 äquivalent): | | |
|---|---|---|
| a) | 10,2 g MgO 18,7 g Ca(OH)$_2$ | (1,5fach äquivalent bezogen auf die Rohproduktsäurezahl) |
| b) | 17,8 g Hydrotalcit 19,7 g Ca(OH)$_2$ | (1,5fach äquivalent bezogen auf die Rohproduktsäurezahl) |

EP 0 502 917 B1

| Kombinationen: | | |
|---|---|---|
| a) | 28,5 g MgO 60 min.<br>+ 20,1 g Ca(OH)$_2$ 120 min. | (2fach äquivalent bezogen auf die Säurezahl des Rohproduktes)<br>(1,5fach äquivalent bezogen auf die Säurezahl nach 60 min.) |
| b) | 47,5 g Hydrotalcit 60 min.<br>+ 8,0 g Ca(OH)$_2$ 120 min. | (2fach äquivalent bezogen auf die Säurezahl des Rohproduktes)<br>(1,5fach äquivalent bezogen auf die Säurezahl nach 60 min.) |

Nach 60, 120 und 180 Minuten wurde jeweils 1/3 der Produktmenge entnommen und mittels Druckfilternutsche filtriert. Die Ergebnisse bezüglich des Inhibitorgehaltes, der Säure- und der Farbzahl sind in der Tabelle 2 zusammengefaßt.

## Tabelle 2

### Inhibitorgehalte in Abhängigkeit vom Neutralisationsmittel und von der Neutralisationszeit

Ausgangsprodukt: Trimethylolpropan + 3 EO-triacrylat

Säurezahl: 38 mg KOH/g

Inhibitor: 1920 ppm 2,5-Di-tert.-butylhydrochinon (DTBHQ)

8

| Temperatur: 80 °C Druck: 50 mbar | Neutrali-sationszeit min. | DTBHQ ppm | Säure-zahl mg KOH/g | Farb-zahl Gardner |
|---|---|---|---|---|
| 49,9 g Ca(OH)2 | 60 | kl. 10 | 0 | kl. 1 |
| (2fach äquival.) | 120 | kl. 10 | 0 | 2 |
| | 180 | kl. 10 | 0 | 3 |
| 37,8 g CaO | 60 | 1050 | 3,9 | kl. 1 |
| (2fach äquival.) | 120 | 750 | 1,7 | 1 - 2 |
| | 180 | kl. 10 | 0,1 | 1 - 2 |
| Kombination | | | | |
| 27,2 MgO (2fach äquival. bez. auf | 60 | 1860 | 15,7 | kl. 1 |
| SZ) + 15,5 g Ca(OH)2 (1,5fach äquival. | 120 | 1450 | 8,1 | kl. 1 |
| bez. auf SZ nach 60 min. | 180 | 1200 | 7,1 | kl. 1 |
| Kombination | | | | |
| 45,2 Hydrotalcit (2fach äquival. bez. | 60 | 850 | 14,1 | kl. 1 |
| auf SZ) + 14,0 g Ca(OH)2 (1,5fach | 120 | 810 | 5,5 | 1 |
| äquival. bez. auf SZ nach 60 min. | 180 | 800 | 4,8 | 1 |
| Mischung: 1:1 äquival. | | | | |
| 18,7 g Ca(OH)2 | 60 | 470 | 3,4 | kl. 1 |
| 10,2 g MgO | 120 | 160 | 3,1 | kl. 1 |
| (1,5fach äquival.) | 180 | 160 | 2,6 | kl. 1 |
| Mischung: 1:1 äquival. | | | | |
| 18,7 g Ca(OH)2 | 60 | 1370 | 7,8 | kl. 1 |
| 17,0 g Hydrotalcit | 120 | 970 | 3,4 | 1 |
| (1,5fach äquival.) | 180 | 690 | 1,8 | 1 |

Beispiel 3

1,0 kg eines Veresterungsproduktes (35 g p-Toluolsulfonsäure; 1,81 g 2,5-Di-tert.-butylhydrochinon; 38,5 g Acrylsäure; 924,7 g Neopentylglykol + 2 PO-diacrylat; Säurezahl: 40 mg KOH/g) wurde in einen 2-Liter-Reaktor eingewogen und bei einer Temperatur von 80 °C und einem Druck von 50 mbar unter Durchleiten von 20 l Luft/h neutralisiert. Die Mengen an Neutralisationsmitteln betrug dabei:

| Ca(OH)₂ | 26,2 g; | 39,9 g; | 52,4 g |
|---------|---------|---------|--------|
| Ca O    | 19,9 g; | 29,8 g; | 39, 7 g |

Nach 1, 2 und 3 Stunden Neutralisationszeit wurde jeweils 1/3 der Produktmenge entnommen und mittels Druckfilternutsche filtriert. Die Ergebnisse bezüglich Säurezahl, Inhibitorgehalt und Farbzahl sind in Tabelle 3 zusammengefaßt.

## Tabelle 3

Inhibitorgehalte in Abhängigkeit von der Neutralisationsmittelmenge und von der Zeit

Ausgangsprodukt: Neopentylglykol + 2 PO-diacrylat

Säurezahl: 40 mg KOH/g

Inhibitor: 1810 ppm 2,5-Di-tert.-butylhydrochinon (DTBHQ)

Gardner Farbzahl: 1

| Temperatur: 80 °C Druck: 50 mbar | Neutralisationszeit min. | DTBHQ ppm | Säurezahl mg KOH/g | Farbzahl Gardner |
|---|---|---|---|---|
| 26,2 g Ca(OH)2 1fach äquival. | 1 | 1020 | 9,2 | kl. 1 |
| | 2 | 830 | 6,7 | kl. 1 |
| | 3 | 770 | 5,7 | kl. 1 |
| 39,3 g Ca(OH)2 1,5fach äquival. | 1 | 890 | 3,6 | kl. 1 |
| | 2 | 680 | 2,5 | kl. 1 |
| | 3 | 600 | 1,8 | kl. 1 |
| 52,4 g Ca(OH)2 2fach äquival. | 1 | kl. 20 | 0,2 | kl. 1 |
| | 2 | kl. 20 | 0 | 2 |
| | 3 | kl. 20 | 0 | 3 |
| 19,9 g CaO 1fach äquival. | 1 | 1810 | 15,7 | kl. 1 |
| | 2 | 1450 | 8,1 | kl. 1 |
| | 3 | 1200 | 7,1 | kl. 1 |
| 29,8 g CaO 1,5fach äquival. | 1 | 1440 | 11,4 | kl. 1 |
| | 2 | 1210 | 6,5 | kl. 1 |
| | 3 | 940 | 4,8 | kl. 1 |
| 39,7 g CaO 2fach äquival. | 1 | 1490 | 5,6 | kl. 1 |
| | 2 | 1200 | 2,6 | kl. 1 |
| | 3 | 890 | 1,6 | kl. 1 |

Beispiel 4

1,8 kg eines Veresterungsproduktes (analog Beispiel 2) wurden in einen 2-Liter-Reaktor eingewogen und bei 80 °C und 50 mbar unter Durchleiten von 20 l Luft/h mit 90,3 Ca(OH)$_2$ neutralisiert.

Nach 10, 20, 30, 40, 50, 60, 120, 180 und 1440 Minuten wurden jeweils ca. 200 g an Produkt entnommen und mittels Druckfilternutsche filtriert. Die Ergebnisse bezüglich Säurezahl, Inhibitorgehalt und Farbzahlen sind in der Tabelle 4 aufgeführt.

## Tabelle 4

Inhibitorgehalte in Abhängigkeit von der Neutralisationszeit

Ausgangsprodukt: Trimethylolpropan + 3 EO-triacrylat

Säurezahl: 38 mg KOH/g

Inhibitor: 1920 ppm 2,5-Di-tert.-butylhydrochinon (DTBHQ)

Gardner Farbzahl: 1

| Temperatur: 80 °C Druck: 50 mbar | Neutrali-sationszeit min. | DTBHQ ppm | Säure-zahl mg KOH/g | Farb-zahl Gardner |
|---|---|---|---|---|
| 90,3 g Ca(OH)2 2fach äquvial. | 10 | 830 | 0,3 | kl. 1 |
| | 20 | 610 | 0 | kl. 1 |
| | 30 | 200 | 0 | kl. 1 |
| | 40 | kl. 10 | 0 | kl. 1 |
| | 50 | kl. 10 | 0 | 1 |
| | 60 | kl. 10 | 0 | 1 |
| | 120 | kl. 10 | 0 | 2 |
| | 180 | kl. 10 | 0 | 3 |
| | 1440 | kl. 10 | 0 | 4 |

## Patentansprüche

1. Verfahren zum teilweisen oder vollständigen Austausch der Radikalinhibitoren (Herstellungsinhibitor) aus ihren Abmischungen mit radikalisch reaktiven, insbesondere polymerisierbaren und/oder vernetzbaren 1- und/oder mehrfach olefinisch ungesättigten Verbindungen gegen nach Art und Menge frei bestimmbare radikalische Inhibitoren oder Inhibitor-Systeme (Applikationsinhibitor), dadurch gekennzeichnet, daß man von radikalisch reaktiven Abmischungen ausgeht, die als Herstellungsinhibitor entsprechende Verbindungen des Phenoltyps mit zur Salzbildung befähigten Hydroxylgruppen enthalten, dieses Einsatzgemisch als Flüssigphase einer Behandlung mit festen Oxiden, Carbonaten und/oder Hydroxiden der Alkali- und/oder Erdalkalimetalle, die auch in Abmischung mit weiteren zur Salzbildung befähigten basischen Metalloxidverbindung, wie Hydrotalcit, vorliegen können, unterwirft und dabei in vorbestimmbarer Weise den Herstellungsinhibitor ganz oder teilweise an die Feststoffphase bindet, die gebildete Feststoffphase von der Flüssigphase abtrennt und in dieser Flüssigphase den Gehalt an Applikationsinhibtor nach Art und Menge geregelt einstellt.

**2.** Verfahren nach Anspruch 1, dadurch gekennzeichnet, daß man radikalisch reaktive Stoffgemische einsetzt, die auf destillativem Weg nicht oder nicht befriedigend von ihrem Gehalt an Herstellungsinhibitor befreit werden können.

**3.** Verfahren nach Ansprüchen 1 und 2, dadurch gekennzeichnet, daß man reaktive Ausgangsmaterialien dem Inhibitoraustausch unterwirft, die Phenol- und/oder Hydrochinonverbindungen, insbesondere alpha-substituierte Hydrochinone als Herstellungsinhibitor enthalten.

**4.** Verfahren nach Ansprüchen 1 bis 3, dadurch gekennzeichnet, daß man den Inhibitoraustausch an Stoffgemischen durchführt, die Dialkyl-substituierte Hydrochinone und insbesondere 2,5-Di-tert.-butylhydrochinon als Herstellungsinhibitor enthalten.

**5.** Verfahren nach Ansprüchen 1 bis 4, dadurch gekennzeichnet, daß man radikalisch stark inhibierende Herstellungsinhibitoren gegen nach Art und/oder Menge schwächer inhibierende Applikationsinhibitoren austauscht.

**6.** Verfahren nach Ansprüchen 1 bis 5, dadurch gekennzeichnet, daß man Herstellungsinhibitoren begrenzter physiologischer Verträglichkeit gegen physiologisch besser verträgliche Applikationsinhibitoren austauscht, wobei als physiologisch verträglicher Applikationsinhibitor insbesondere Vitamin E gewählt wird.

**7.** Verfahren nach Ansprüchen 1 bis 6, dadurch gekennzeichnet, daß man radikalisch inhibierte Reaktivsysteme auf Basis von (Meth)acrylatestern mehrwertiger Alkohole, insbesondere 2- bis 4-wertiger Alkohole dem Inhibitoraustausch unterwirft.

**8.** Verfahren nach Ansprüchen 1 bis 7, dadurch gekennzeichnet, daß man als Feststoffphase zur Bindung des Herstellungsinhibitors die Oxide und/oder Hydroxide von Calcium und/oder Magnesium einsetzt, wobei die wenigstens anteilsweise Mitverwendung entsprechender Calciumverbindungen bevorzugt ist.

**9.** Verfahren nach Ansprüchen 1 bis 8, dadurch gekennzeichnet, daß man die Bindung des Herstellungsinhibitors an der bevorzugt feinteiligen Feststoffphase im Temperaturbereich von etwa 60 bis 100 °C, insbesondere im Bereich von etwa 70 bis 90 °C bei einer Behandlungsdauer von zweckmäßigerweise nicht mehr als etwa 3 Stunden durchführt, wobei eine Dauer der Behandlung im Bereich von etwa 5 Minuten bis 1 Stunde bevorzugt sein kann.

**10.** Verfahren nach Ansprüchen 1 bis 9, dadurch gekennzeichnet, daß man mit wenigstens weitgehend wasserfreien Einsatzmaterialien arbeitet und bevorzugt auch während der Behandlung der Flüssigphase mit den feinteiligen Feststoffen wenigstens anteilsweise unter verringertem Druck und Abziehen des entstehenden Neutralisationswassers arbeitet, wobei in dieser Stufe das Arbeiten im Druckbereich von etwa 1 bis 150 mbar bevorzugt sein kann.

**11.** Verfahren nach Ansprüchen 1 bis 10, dadurch gekennzeichnet, daß man mit Polymerisations- bzw. Vergelungs-gefährdeten olefinisch ungesättigten Einsatzmaterialien arbeitet, die auch in Abmischung mit Lösungs- und/oder Verdünnungsmitteln vorliegen können und zusätzlich saure Verunreinigungen wie Restsäureanteile aus der Kondensation, saure Katalysatoren und dergleichen enthalten.

**12.** Verfahren nach Ansprüchen 1 bis 11, dadurch gekennzeichnet, daß man die Bindung des Herstellungsinhibitors mit einer Trockenneutralisation des radikalisch reaktiven Systems verbindet.

**13.** Verfahren nach Ansprüchen 1 bis 12, dadurch gekennzeichnet, daß man das feinpulvrige Binde- und Neutralisationsmittel in wenigstens stöchiometrischer Menge, vorzugsweise in beschränktem Überschuß einsetzt, wobei die Verwendung von Mengen im Bereich des etwa 1,3- bis 2,5-fachen der stöchiometrischen Menge bevorzugt sein kann.

**14.** Verfahren nach Ansprüchen 1 bis 13, dadurch gekennzeichnet, daß man zur praktisch vollständigen Entfernung des Herstellungsinhibitors trockenes pulverförmiges Calciumhydroxid einsetzt.

**Claims**

1. A process for the partial or complete exchange of the free radical inhibitors (preparation inhibitor) from their admixtures with free radical-reactive - and especially polymerizable and/or cross-linkable - olefinically mono- and/or polyunsaturated compounds against free radical inhibitors or inhibitor systems (application inhibitor), the kind and amount of which are freely determinable, characterized in that admixtures reactive via free radicals are employed which contain, as the preparation inhibitor, appropriate compounds of the phenol type comprising hydroxyl groups capable of forming salts, this feedstock mixture as a liquid phase is subjected to a treatment with solid oxides, carbonates and/or hydroxides of the alkali metals and/or alkaline earth metals, which may also be present in admixture with further basic metal oxide compounds capable of forming salts, such as hydrotalcite, thereby in a pre-determinable manner binding all or part of the preparation inhibitor to the solid phase, the resulting solid phase is separated from the liquid phase, and the content of the application inhibitor in this liquid phase is adjusted with respect to kind an amount thereof.

2. The process according to claim 1, characterized in that free radical-reactive substance mixtures are employed, from which the preparation inhibitors contained therein cannot be removed or cannot be satisfactorily removed by way of a distillation.

3. The process according to claims 1 and 2, characterized in that reactive starting materials are subjected to the inhibitor exchange, which starting materials contain phenol and/or hydroquinone compounds, and especially α-substituted hydroquinones, as the preparation inhibitor.

4. The process according to claims 1 to 3, characterized in that the inhibitor exchange is carried out with substance mixtures containing dialkyl-substituted hydroquinones, and especially 2,5-di-tert.-butylhydroquinone as the preparation inhibitor.

5. The process according to claims 1 to 4, characterized in that preparation inhibitors having strong free radical-inhibiting action are exchanged for application inhibitors having a weaker free radical-inhibiting action with respect to kind and/or quantity thereof.

6. The process according to claims 1 to 5, characterized in that preparation inhibitors having a limited physiological compatibility are exchanged for physiologically better compatible application inhibitors, wherein vitamin E is chosen as the physiologically compatible application inhibitor.

7. The process according to claims 1 to 6, characterized in that free radical-inhibited reactive systems based on (meth)acrylate esters of polyhydric alcohols, and especially of di- to tetrahydric alcohols, are subjected to the inhibitor exchange.

8. The process according to claims 1 to 7, characterized in that the oxides and/or hydroxides of calcium and/or magnesium are employed as the solid phase for binding the preparation inhibitor, wherein the at least partial concomitant use of corresponding calcium compounds is preferred.

9. The process according to claims 1 to 8, characterized in that the process of binding the preparation inhibitor to the preferably finely divided solid phase is effected within a temperature range of from about 60 °C to 110 °C, especially within the range of from about 70 °C to 90 °C, with a duration of the treatment of appropriately not more than about 3 hours, wherein a duration of the treatment within the range of from about 5 minutes to 1 hour may be preferred.

10. The process according to claims 1 to 9, characterized in that at least largely anhydrous feedstock materials are employed and that preferably also in the course of the treatment of the liquid phase with the finely divided solids a reduced pressure is applied, thereby to at least partially withdraw the water resulting from the neutralization, wherein in this step it may be preferred to employ a pressure within the range of from 1 to 150 mbar.

11. The process according to claims 1 to 10, characterized in that olefinically unsaturated feedstock materials are treated which are susceptible to polymerization or gelling and which may also be present in an admixture with solvents and/or diluents and may additionally contain acidic contaminations and residual amounts of acids from condensations, acidic catalysts and the like.

12. The process according to claims 1 to 11, characterized in that the process of binding the preparation inhibitor is realized in combination with a dry neutralization of the free radical-reactive system.

13. The process according to claims 1 to 12, characterized in that the finely powdered binding and neutralizing agent is employed in an at least stoichiometric amount, preferably in a limited excess, wherein the use of amounts within the range of from about 1.3 times to about 2.5 times the amount as stoichiometrically required may be preferred.

14. The process according to claims 1 to 13, characterized in that dry powdery calcium hydroxide is employed for effecting the virtually complete removal of the preparation inhibitor.

**Revendications**

1. Procédé pour l'échange partiel ou complet des inhibiteurs radicalaires (inhibiteur de préparation) à partir de leurs mélanges avec des composés à radicaux réactifs, particulièrement polymérisables et/ou réticulables, une ou plusieurs fois oléfiniquement insaturés contre des inhibiteurs ou des systèmes d'inhibiteurs (inhibiteur d'application) radicalaires librement déterminables en type et en quantité, caractérisé en ce qu'on part de mélanges à radicaux réactifs, qui contiennent des composés correspondants comme inhibiteur de préparation du type phénol avec des groupes hydroxyle aptes à la formation de sel, ce mélange d'application est soumis comme phase liquide à un traitement avec des oxydes, carbonates et/ou hydroxydes solides de métaux alcalins et/ou alcalino-terreux, qui peuvent être présents aussi en mélange avec d'autres composés oxydo-métalliques basiques aptes à la formation de sels, comme l'hydrotalcite et en outre il combine de manière prédéterminable l'inhibiteur de préparation totalement ou partiellement à la phase solide, la phase solide formée se sépare de la phase liquide et ajuste de manière contrôlée la teneur en inhibiteur d'application selon le type et la quantité.

2. Procédé selon la revendication 1, caractérisé en ce qu'on met en oeuvre des mélanges de matières à radicaux réactifs, qui ne peuvent pas ou de manière non satisfaisante être libérés de leur contenu en inhibiteur de préparation par distillation.

3. Procédé selon les revendications 1 à 3, caractérisé en ce qu'on soumet à l'échange d'inhibiteur, des matières d'origine réactives, qui contiennent des composés de phénol et/ou d'hydroquinone, en particulier d'hydroquinones α substituées comme inhibiteur de préparation.

4. Procédé selon les revendications 1 à 3, caractérisé en ce qu'on réalise l'échange d'inhibiteurs sur des mélanges de matières, qui contiennent des hydroquinones dialkyl substituées et en particulier de l'hydroquinone 2,5 di-ter-butylique comme un inhibiteur de préparation.

5. Procédé selon les revendications 1 à 4, caractérisé en ce qu'on échange des inhibiteurs de préparation à forte inhibition radicalaire contre des inhibiteurs d'application à plus faible inhibition en type et/ou en quantité.

6. Procédé selon les revendications 1 à 5, caractérisé en ce qu'on échange des inhibiteurs de préparation de comptabilité physiologique limitée contre des inhibiteurs d'application physiologique mieux compatibles, parmi lesquels comme inhibiteur d'application physiologiquement compatible on choisit en particulier la vitamine E.

7. Procédé selon les revendications 1 à 6, caractérisé en ce qu'on soumet des systèmes réactifs radicalement inhibés, à base d'esters de (méth)acrylates d'alcools plurivalents, en particulier d'alcools di à tétravalents, à l'échange d'inhibiteurs.

8. Procédé selon les revendications 1 à 7, caractérisé en ce qu'on met en oeuvre comme phase solide pour la combinaison de l'inhibiteur de préparation les oxydes et/ou hydroxydes de calcium et/ou de magnésium, parmi lesquels on préfère au moins en proportion la co-utilisation de composés de calcium adéquats.

9. Procédé selon les revendications 1 à 8, caractérisé en ce qu'on réalise la combinaison de l'inhibiteur de préparation sur la phase solide de préférence finement divisée dans la gamme de température de 60 à 100°C, environ en particulier dans la gamme de 70 à 90°C environ avec une durée de traitement normalement non supérieure à environ 3 heures, alors qu'une durée de traitement dans la plage de 5 minutes environ à 1 heure peut être préférée.

10. Procédé selon les revendications 1 à 9, caractérisé en ce qu'on travaille avec des matières d'utilisation au moins largement anhydres et qu'on opère de préférence aussi pendant le traitement de la phase liquide avec les matières solides finement divisées au moins partiellement sous pression réduite et en retirant l'eau de neutralisation résultante, tout en pouvant préférer dans cette étape opérer dans le domaine de pression compris entre 1 et 150 mbars.

11. Procédé selon les revendications 1 à 10, caractérisé en ce qu'on travaille avec des matières d'utilisation oléfiniquement insaturées menacées de polymérisation ou de gélification, qui peuvent être présentes aussi en mélange avec des solvants et/ou des diluants et contiennent en plus des impuretés acides comme des fractions d'acide résiduelles provenant de la condensation, de catalyseurs acides et analogues.

12. Procédé selon les revendications 1 à 11, caractérisé en ce qu'on connecte la combinaison de l'inhibiteur de préparation avec une neutralisation à sec du système à radicaux réactifs.

13. Procédé selon les revendications 1 à 12, caractérisé en ce qu'on met en oeuvre l'agent de combinaison et de neutralisation finement pulvérulent en quantité au moins stoechiométrique, de préférence en excès limité, tout en pouvant préférer utiliser des quantités au niveau de 1,3 fois à 2,5 fois environ la quantité stoechiométrique.

14. Procédé selon les revendications 1 à 13, caractérisé en ce qu'on met en oeuvre de l'hydroxyde de calcium pulvérulent sec pour l'élimination pratiquement complète de l'inhibiteur de préparation.